Europäisches Patentamt

European Patent Office

Office européen des brevets

(19)

(11) Veröffentlichungsnummer: **0 031 472**
**B1**

(12) EUROPÄISCHE PATENTSCHRIFT

(45) Veröffentlichungstag der Patentschrift:
**27.07.83**

(21) Anmeldenummer: **80107446.9**

(22) Anmeldetag: **28.11.80**

(51) Int. Cl.³: **B 01 J 23/74,** C 07 C 1/04,
C 07 C 9/04

(54) Verfahren zur Herstellung von Methanisierungskatalysatoren.

(30) Priorität: **29.12.79 DE 2952683**

(43) Veröffentlichungstag der Anmeldung:
**08.07.81 Patentblatt 81/27**

(45) Bekanntmachung des Hinweises auf die Patenterteilung:
**27.07.83 Patentblatt 83/30**

(84) Benannte Vertragsstaaten:
**BE DE FR GB NL**

(56) Entgegenhaltungen:
**DE-A-2 231 316**
**DE-A-2 231 367**
**DE-A-2 261 634**
**DE-A-2 449 587**
**DE-A-2 621 314**
**DE-A-2 631 901**
**DE-A-2 651 567**
**DE-A-2 807 422**
**DE-A-2 816 035**
**FR-A-863 311**
**FR-A-1 523 687**
**US-A-3 933 883**

(73) Patentinhaber: **Ruhrchemie Aktiengesellschaft,
Bruchstrasse 219, D-4200 Oberhausen 13 (DE)**

(72) Erfinder: **Horn, Gerhard, Dr. Dipl.-Chem.,
Bunsenstrasse 19, D-4200 Oberhausen 13 (DE)**
Erfinder: **Frohning, Dieter, Dr. Dipl.-Chem., Elsenbruch
la, D-4200 Oberhausen 13 (DE)**

(74) Vertreter: **Reichelt, Karl-Heinz, Dr., m. Br. Ruhrchemie
Aktiengesellschaft Abt. PLD Postfach 13 01 60,
D-4200 Oberhausen 13 (DE)**

# 0 031 472

## Verfahren zur Herstellung von Methanisierungskatalysatoren

Gegenstand der vorliegenden Erfindung ist ein Verfahren zur Herstellung von Methanisierungskatalysatoren, die Nickel und Kobalt als aktive Bestandteile in Kombination mit MgO als Promotor und einen Träger enthalten. Die erhaltenen Katalysatoren sind besonders geeignet für die Hydrierung von Kohlenmonoxid zu vorwiegend Methan in einem Fließbett- oder Wirbelschicht-Verfahren.

Die absehbare Verknappung von natürlichem Erdgas hat zur Entwicklung verschiedener Verfahren zur katalytischen Hydrierung von Kohlenmonoxid geführt. Derartige Verfahren haben die Erzeugung eines synthetischen, mit natürlichem Erdgas in bezug auf Zusammensetzung und Verwendbarkeit weitgehend gleichen und damit austauschbaren Gases (Substitute Natural Gas oder SNG) zum Ziel. Natürliches Erdgas enthält vielfach neben Methan noch Ethan, Propan oder auch Butan in Anteilen bis zu insgesamt etwa 0,1 bis 5,0 Vol.-%, bezogen auf den Gehalt an Methan. Diese Kohlenwasserstoffe erhöhen den Heizwert des Gases und sind daher im gewissen Umfange erwünscht, beeinflussen jedoch die Dichte und die Brenneigenschaften des Gases, so daß ihr Gehalt innerhalb gewisser Grenzen limitiert werden muß.

Die Verfahren zur katalytischen Hydrierung von Kohlenmonoxid zu Methan unterscheiden sich vor allem durch die Maßnahmen, die zur Abführung der bei der Methanisierung frei werdenden Reaktionswärme getroffen werden. In den meist mehrstufigen Verfahren sind die Katalysatoren in ruhender Schüttung angeordnet. Neuere Verfahrensentwicklungen wenden die Wirbelschicht-Technik für die Methanisierung an, da auf diesem Wege besonders hohe Raum-Zeit-Ausbeuten erzielt werden und zugleich wegen des guten Wärmeüberganges die thermische Beanspruchung des Katalysators in Grenzen gehalten wird. Beispiele für die katalytische Hydrierung von Kohlenmonoxid zu vorwiegend Methan unter Anwendung der Wirbelschicht-Technik finden sich in den DE-A-2 449 587, 2 651 567 und 2 807 422.

Die Hydrierung von Kohlenmonoxid zu vorwiegend Methan wird von Nickel, Ruthenium, Kobalt oder Eisen katalysiert. Katalysatoren für technische Anforderungen enthalten fast ausschließlich Nickel als aktive Hauptkomponente, wobei der Nickel-Gehalt üblicherweise 30 bis 40% des Gesamtgewichtes der Katalysatoren beträgt. Die Verwendung von Kobalt als aktive Hauptkomponente für Methanisierungskatalysatoren ist ohne Bedeutung, da Kobalt ebenso wie Eisen im Vergleich zu Nickel wesentliche Nachteile aufweist, die vor allem in der Tendenz zur vermehrten Abscheidung von Kohlenstoff bestehen. Die Verwendung von Ruthenium, das ausgezeichnete Eigenschaften für die Hydrierung von Kohlenoxiden besitzt, hat bisher keinen Eingang in die technische Praxis gefunden.

Neben hydrieraktiven Metallen enthalten Methanisierungskatalysatoren üblicherweise Zusätze an schwer reduzierbaren Oxiden, die sowohl als elektronische bzw. strukturelle Promotoren oder auch als Trägermaterialien bzw. Bestandteile von Trägermaterialien Verwendung finden.

Es ist bekannt, Nickel und Kobalt gemeinsam auf Trägern als Katalysator für die Hydrierung von Kohlenmonoxid zu verwenden. So wird in der DE-A-2 621 314 die Herstellung eines Nickel und Kobaltoxid enthaltenden Katalysatorvorprodukts durch Fällung beschrieben. Der aus dem Vorprodukt gewonnene Katalysator enthält mehr als 40 Gew.-% Nickeloxid und Kobaltoxid und wird in Form eines Katalysator-Festbettes für die Methanisierung verwendet.

Die DE-A-2 631 901 betrifft einen Katalysator für die Methanisierung nach dem Festbett-Verfahren, der neben Nickel als aktiver Hauptkomponente auch Kobalt, Eisen, Kupfer, Magnesium, Zink, Aluminium und Chrom enthalten kann. Dieser Katalysator wird hergestellt durch gemeinsame Fällung der Komponenten als Silicate, die eine natürliche Mineralien (Serpentin) vergleichbare Zusammensetzung aufweisen. Außer den Metallsilicaten enthält dieser Katalysator Tonmineralien. Der Gehalt an Nickel bzw. Nickel und Kobalt im Katalysator beträgt 65 Gew.-%. Angaben, ob und in welcher Weise die Eigenschaften des Katalysators durch Hinzufügen von Kobalt und/oder Magnesium beeinflußt werden, finden sich in der genannten Druckschrift nicht.

In der FR-A-1 523 687 werden eine Vielzahl sehr allgemeiner qulitativer und quantitativer Hinweise auf die Zusammensetzung von Methanisierungskatalysatoren gegeben. Die Beispiele konzentrieren sich aber ausschließlich auf Katalysatoren, die neben einem Träger lediglich Nickel, Chrom und Magnesium enthalten.

Weitere Verfahren zur Herstellung nickelhaltiger Methanisierungskatalysatoren durch Ausfällung der aktiven Komponenten aus wäßriger Lösung in Gegenwart eines Trägermaterials oder durch gemeinsame Fällung der aktiven Bestandteile und der Trägermaterialien aus ihrer Lösung sind z. B. in den DE-A-2 231 316, 2 231 367 und 2 261 634 beschrieben.

Eine weitere Möglichkeit der Aufbringung von für die Methanisierung katalytisch aktiven Komponenten auf Trägermaterialien besteht in der Imprägnierung oberflächenreicher Träger. So wird in der US-A-3 933 883 ein Methanisierungskatalysator beschrieben, der ein Nickel-Kobalt-Mischoxid auf hochreinem $\gamma$-$Al_2O_3$ enthält und durch Imprägnierung des Trägermaterials mit einer Nickel- und Kobaltsalze enthaltenden Lösung und nachfolgende Calcination erhalten wurde. Der Katalysator weist eine unzureichende Katalysatorleistung auf und ist nicht für die Methanisierung in der Wirbelschicht geeignet.

Katalysatoren für die Methanisierung in der Wirbelschicht sind in dem Tagungsbericht »Evaluation of Fluidized-Bed Methanation Catalysts«, des »8. Synthetic Pipeline Gas Symposium«, der »American Gas Association«, der »Energy Research and Development Administration« und der »International Gas Union« 1976 in Monroeville/Pennsylvania sowie in der DE-A-2 449 587 beschrieben worden. Danach werden feinteilige Katalysatoren aus Gemischen von Nickeloxid mit Oxiden des Chroms, Molybdäns und Wolframs bzw. von Kobalt mit Chrom-, Molybdän- und Wolframoxiden auf Aluminiumoxid als Träger für die gleichzeitige Konvertierung und Methanisierung in der Wirbelschicht eingesetzt. Die Herstellung der Katalysatoren ist nicht beschrieben. Überdies sind die mit den angeführten Katalysatoren erreichten Standzeiten von 25 bis 26 Tagen unbefriedigend.

In der DE-A-2 816 035 wird ein Fließbettkatalysator zur Herstellung von synthetischem Erdgas beschrieben. Der Katalysator wird durch mechanisches Vermischen von Nickeloxid mit Trägermaterial und hydraulischem Zement als Bindemittel in angefeuchtetem Zustand, anschließendes Pressen zu Formkörpern, die zunächst thermisch behandelt und danach auf eine Teilchengröße von 40 bis 350 $\mu$m zerkleinert werden, hergestellt. Die Herstellung dieses Katalysators ist offensichtlich sehr aufwendig. Auch mit diesem Katalysator können keine befriedigenden Standzeiten erzielt werden.

Für Wirbelschicht-Verfahren werden an Katalysatoren spezielle Anforderungen gestellt. Zusätzliche Forderungen ergeben sich bei der katalytischen Hydrierung von Kohlenmonoxid, die vorwiegend zu Methan führt. Aufgrund der für Reaktionen in der Wirbelschicht typischen hohen Gasgeschwindigkeiten, die kurze Verweilzeiten der Reaktionsteilnehmer am Katalysator zur Folge haben, müssen die Katalysatoren hohe Aktivität aufweisen, um eine rasche Einstellung des Reaktionsgleichgewichts zu gewährleisten.

Zur Gewährleistung eines einwandfreien Fluidisierungsverhaltens und damit eines störungsfreien technischen Betriebes sind ferner bestimmte Anforderungen an die mechanischen Eigenschaften des Katalysators zu stellen, die sich vor allem auf seine Korngrößenverteilung und auf seine Dichte beziehen. Ein wesentliches Merkmal für die Beurteilung eines Wirbelschicht-Katalysators liegt außerdem in seiner mechanischen Beständigkeit gegen Abrieb. Der Anteil an Feinstmaterial, der aus betriebstechnischen Gründen ausgeschleust werden muß, soll weniger als 1 Gew.-% der Katalysatormasse je Betriebstag betragen, um die Verluste in tragbaren Grenzen zu halten (vgl. z. B. A. Anderlohr, K. Hedden: GWF Gas, Erdgas 118, 422 [1977]).

Für die Herstellung von Methanisierungskatalysatoren für Wirbelschicht-Verfahren ist die Katalysatorherstellung durch Fällung aus mehreren Gründen nachteilig. Die gleichmäßige Abscheidung der aktiven Komponenten auf einem Träger erfordert große Sorgfalt und ist vielfach schwierig zu reproduzieren. Der Katalysator-Vorläufer bzw. der fertige Katalysator wird gewöhnlich in regelmäßiger oder unregelmäßiger Form erhalten, nicht jedoch in der Korngrößenverteilung, wie sie für die Verwendung im Wirbelschicht-Verfahren erforderlich ist. Die Maßnahmen zur Einstellung der gewünschten Korngrößenverteilung führen unweigerlich zu einem Zwangsanfall an Fein- und Grob-Anteilen, der in den Herstellungsgang wieder eingefügt oder anderweitig verwertet bzw. als Verlust angesehen werden muß. Besonders bei Katalysatoren mit hohen Gehalten an den teuren aktiven Komponenten Nickel und Kobalt können hieraus erhebliche wirtschaftliche Nachteile entstehen.

Zur Erzielung einer technisch und wirtschaftlich befriedigenden Betriebszeit muß der Katalysator auch bei hohen Reaktionstemperaturen, d. h. etwa zwischen 300 und 600°C, ausreichend beständig sein.

Der relativ hohe Nickel- und Kobalt-Gehalt der durch Fällung hergestellten Katalysatoren, er beträgt etwa 30 bis 60 Gew.-% der Katalysatormasse, führt zu hohen Kosten bei der Verwendung dieser Metalle in Methanisierungsverfahren. Eine Senkung der Konzentrationen der aktiven Komponenten Nickel und Kobalt auf einen wirtschaftlich günstigeren Wert von z. B. weniger als 30%, bezogen auf den Gesamtkatalysator, führt andererseits bei Fällungskatalysatoren zu einem deutlichen Rückgang der Aktivität und der nutzbaren Betriebszeit.

Zusammengefaßt ergibt sich somit, daß ein für die Methanisierung von Kohlenmonoxid in einem Fließbett- oder Wirbelschicht-Verfahren geeigneter Katalysator insbesondere folgenden Anforderungen genügen muß:

Er muß bei möglichst niedriger Konzentration der aktiven Komponenten hohe Aktivität besitzen, ferner hervorragende mechanische Stabilität und einwandfreies Fluidisierungsverhalten aufweisen und gegen hohe Temperaturen beständig sein. Seine Herstellung soll einfach und wirtschaftlich sein und reproduzierbar die gewünschten Katalysatoreigenschaften sicherstellen.

Schließlich soll das durch Hydrierung von Kohlenmonoxid entstehende vorwiegend Methan enthaltende Gasgemisch in seiner Zusammensetzung dem natürlichen Erdgas ähnlich und weitgehend frei von nicht umgesetztem Kohlenmonoxid sein. Zur Herabsetzung der Flammgeschwindigkeit wird außerdem ein möglichst niedriger Wasserstoffgehalt angestrebt. Ein Anteil an Ethan, Propan oder Butan im Bereich von insgesamt 0,1 bis 5 Vol.-%, bezogen auf Methan, bewirkt eine Erhöhung des Heizwertes des methanreichen Gases und ist somit erwünscht.

Überraschenderweise werden die vorgenannten Aufgaben durch Methanisierungskatalysatoren gelöst, die aus Kobalt, Nickel und einem Trägermaterial bestehen und zusätzlich Magnesiumoxid enthalten, das atomare Verhältnis von Nickel, Kobalt und Magnesium von 1 : 0,05 bis 0,5 : 0,05 bis 0,5 beträgt, das Trägermaterial mit thermisch zersetzbaren Salzen des Nickels, Kobalts und Magnesiums

0 031 472

imprägniert wird und die aufgetragenen Salze durch Trocknung auf dem Träger fixiert und durch nachfolgende thermische Behandlung in die Metalloxide überführt werden und darauf bei 350 bis 500° C mit reinem Wasserstoff oder Wasserstoff enthaltenden Gasen reduziert wird.

Die nach der thermischen Behandlung erhaltenen Katalysatorvorläufer, in denen die Metalle als Oxide vorliegen, enthalten 5 − 18 Gew.-% Nickel, 0,25 − 9,0 Gew.-% Kobalt und 0,10 − 3,73 Gew.-% Magnesium, jeweils bezogen auf die gesamte Katalysatormasse einschließlich Träger. Die Kombination von Nickel, Kobalt und Magnesium auf geeigneten Trägermaterialien zeichnet sich für Methanisierungskatalysatoren in mehrfacher Hinsicht als vorteilhaft und den binären Kombinationen aus Nickel und Magnesium, Kobalt und Magnesium bzw. Nickel und Kobalt auf Trägern als überlegen aus. Vermutlich liegt bei den Ni − Co − Mg-Kombinationen durch Mischkristallbildung eine besonders gleichmäßige Verteilung der drei Bestandteile untereinander vor. Die nahezu statistische Verteilung von Nickel, Kobalt und Magnesium wird als eine der Ursachen für die überdurchschnittliche Leistungsfähigkeit der erfindungsgemäß hergestellten Katalysatoren angesehen.

Die bei der thermischen Behandlung und unter den Bedingungen der Methanisierung zwischen Nickel, Kobalt und Magnesium stattfindende Bildung von Verbindungen vom Spinelltyp erhöht die Beständigkeit der aktiven Katalysatorbestandteile gegen Rekristallisation, so daß die neuen Katalysatoren lange Betriebszeiten erreichen.

Als Trägermaterialien können Aluminiumoxid, Aluminiumoxid-Siliciumdioxid-Kombinationen, beispielsweise in Form von Aluminiumsilicaten oder Aluminiumoxid-Siliciumdioxid-Mischoxiden oder Siliciumdioxid verwendet werden. Bevorzugt werden als Trägermaterialien $\gamma$-Al$_2$O$_3$ oder Aluminiumoxid-Siliciumdioxid-Kombinationen mit 5 − 30, vorzugsweise 10 − 20 Gew.-% Aluminiumoxid, bezogen auf die Gesamtkombination. Der Katalysator enthält auf 100 Gew.-Teile Nickel — als Metall gerechnet — 300 bis 1850 Gew.-Teile, vorzugsweise 400 − 900 Gew.-Teile Trägermaterial. Die anmeldungsgemäß hergestellten Methanisierungskatalysatoren eignen sich insbesondere für den Einsatz in Fließbett- oder Wirbelschichtverfahren. Im Hinblick auf eine gute Fluidisierbarkeit des Katalysators liegt die Kornverteilung des verwendeten Trägermaterials vorzugsweise in den folgenden Bereichen:

| Korngrößenbereich | Gew.-% des Trägermaterials |
|---|---|
| <32 μm | 10 |
| >32 μm − 63 μm | 10—15 |
| >62 μm − 80 μm | 15—25 |
| >83 μm − 100 μm | 20—40 |
| >100 μm − 125 μm | 30—50 |
| >125 μm − 250 μm | 10—20 |

Für das Fluidisierverhalten des Katalysators ist ferner die Schüttdichte bzw. das Schüttgewicht des Trägermaterials, d. h. das Gewicht von 1 l loser Schüttung (500 − 1200 g/l) von Bedeutung.

Für die erfindungsgemäß hergestellten Katalysatoren werden Trägermaterialien mit Oberflächen von 130 bis 600 m$^2$/g, vorzugsweise von 150 bis 300 m$^2$/g (bestimmt nach der BET-Methode) verwendet. Um die angeführten Konzentrationen der aktiven Komponenten aufbringen zu können, soll das Trägermaterial ein Porenvolumen von mehr als 0,35 cm$^3$/g aufweisen. Für die Verwendung der erfindungsgemäß hergestellten Katalysatoren im Fließbett- oder Wirbelschicht-Verfahren soll das Porenvolumen des Trägermaterials einen Wert von 0,8 cm$^3$/g nicht überschreiten, da sonst in der Regel die Abriebfestigkeit nicht ausreicht. Trägermaterialien, die die geforderten Eigenschaften aufweisen, sind im Handel erhältlich.

Die Herstellung der neuen Katalysatoren erfolgt durch Imprägnierung eines Trägermaterials, das die vorgenannten Eigenschaften aufweist, mit einer Lösung, die thermisch zersetzbare Salze des Nickels, Kobalts und des Magnesiums in dem angegebenen Atomverhältnis und in einer Konzentration enthält, die ausreicht, um nach thermischer Behandlung einen Gehalt von 5 bis 18 Gew.-% Nickel im Katalysator sicherzustellen. Die Imprägnierung des Trägermaterials kann durch Tränkung, durch Sprühtrocknung oder durch ein der Sprühtrocknung ähnliches Verfahren vorgenommen werden.

Für die Imprägnierung der Trägermaterialien kommen Lösungen thermisch zu Oxiden zersetzbarer Verbindungen des Nickels, Kobalts und des Magnesiums, vorzugsweise die Nitrate oder Acetate mit einem Atomverhältnis der Metalle Ni : Co : Mg wie 1 : 0,05 bis 0,5 : 0,05 bis 0,5 zur Anwendung. Die Metallkonzentration der Lösungen beträgt 50 bis 300 g Ni/l. Nicht geeignet sind Nickel-, Kobalt- und Magnesium-Salze, die keine ausreichende Löslichkeit in dem angegebenen Konzentrationsbereich aufweisen, beispielsweise Formiate oder Oxalate.

4

Bei der Imprägnierung durch Tränkung wird das Trägermaterial in die auf 50−100°C erwärmte Nickel, Kobalt und Magnesium enthaltende Lösung eingetragen. Die Konzentration der Lösung soll 80−300 g Nickel pro Liter, vorzugsweise 150−250 g Nickel pro Liter und die Dauer der Tränkung etwa 0,5 bis 3 Stunden betragen bei einer Temperatur der Suspension von 50−100°C. Zweckmäßig wird die Suspension während des Tränkungsvorganges gut durchmischt. Nach beendeter Tränkung trennt man den imprägnierten Träger von der überschüssigen Tränkungslösung, beispielsweise durch Filtration, ab und trocknet ihn bei erhöhter Temperatur, beispielsweise im Bereich von 50−130°C, stationär. Die überschüssige Tränkungslösung, die noch Nickel, Kobalt und Magnesium enthält, kann nach Einstellung der gewünschten Konzentrationen erneut für die Imprägnierung verwendet werden, so daß keine Verluste an den wertvollen Metallsalzen auftreten.

Für die Herstellung des erfindungsgemäßen Katalysators durch Sprühtrocknung wird ebenfalls eine Suspension der oben beschriebenen Trägermaterialien in einer Nickel, Kobalt und Magnesium enthaltenden Salzlösung verwendet. Das Atomverhältnis der in der Tränkungslösung vorliegenden Metalle beträgt wie bei der ersten Ausführungsform Ni : Co : Mg = 1 : 0,05−0,5 : 0,05−0,5. Die Konzentration der Lösung bezüglich Ni liegt im Bereich von 50 bis 280 g Nickel pro Liter, vorzugsweise zwischen 100 und 250 g Nickel pro Liter. Auf 100 Gew.-Teile Nickel werden 300 bis 1850 Gew.-Teile, vorzugsweise 400 bis 900 Gew.-Teile Trägermaterial eingesetzt. Nach Einwirkenlassen der Metallsalzlösung auf den Träger bei 50 bis 100°C über einen Zeitraum von 0,5 bis 2 Stunden wird die Suspension mit Hilfe eines mit hoher Geschwindigkeit rotierenden Zerstäubers verteilt und im Heißluftstrom bei Temperaturen von 90 bis 350°C getrocknet. Gegebenenfalls setzt man der Suspension ein geeignetes Bindemittel, z. B. hydraulischen Zement, hinzu, um die Abriebfestigkeit der auf diesem Wege erhaltenen Katalysatorteilchen zu verbessern, jedoch ist der Zusatz dieser Bindemittel nicht zwingend erforderlich.

Bei einem der Sprühtrocknung verwandten Verfahren wird statt einer Suspension der mit Metallsalzen beladene Träger in Form einer feuchten pastenförmigen Masse eingesetzt und unter mechanischer Zerteilung der feuchten Masse und Aufwirbelung in einem heißen Luftstrom getrocknet. Der Luftstrom besitzt dabei eine Temperatur, die unterhalb der Zersetzungstemperatur der verwendeten Metallsalze zu Oxiden liegt, die jedoch für eine Trocknung ausreichend ist. Bevorzugt werden Temperaturen im Bereich von 90−300°C angewandt.

Zum Einsatz kommen hier ebenfalls Metallsalzlösungen mit einem Atomverhältnis der Metalle Nickel, Kobalt und Magnesium von 1 : 0,05−0,5 : 0,05−0,5. Die Konzentration der Lösung liegt bezüglich Nickel zwischen 160 und 300 g Nickel pro Liter, vorzugsweise 200 bis 250 g Nickel pro Liter. Als Träger werden die oben beschriebenen Materialien in Mengen von 300−1850, vorzugsweise 400−900 Gew.-Teilen Träger auf 100 Gew.-Teile Nickel als Metall gerechnet, verwendet. Die Konzentration der Lösung und die Trägermenge werden so aufeinander abgestimmt, daß eine pastöse bis feucht krümelige Masse erhalten wird, wobei das genannte Verhältnis von Nickel-Einsatzmenge zu Trägermenge einzuhalten ist.

Die Mischung von Metallsalzlösung und Trägermaterial wird 0,5 bis 2 Stunden bei Temperaturen von 50 bis 100°C gemischt. Zur Trocknung wird die feuchte Masse allmählich auf ein Rotorsystem im Heißluftstrom aufgegeben und auf diese Weise zerkleinert und getrocknet. Die getrockneten Partikel des Katalysatorvorproduktes werden vom heißen Luftstrom aus der Trocknungszone transportiert und in einem Zyklon abgeschieden.

Die aus der Imprägnierung von Trägermaterialien durch Tränkung, Sprühtrocknung oder der Sprühtrocknung ähnlichen Verfahren hervorgegangenen Katalysatorvorläufer werden zur Überführung der Metallsalze in Oxide einer thermischen Behandlung unterworfen. Sie wird zwischen 300 und 650°C, vorzugsweise 400 und 600°C, in Gegenwart von Stickstoff oder Luft durchgeführt. Die Zeitdauer für die thermische Behandlung beträgt 0,5 bis 5 Stunden, vorzugsweise 1 bis 3 Stunden. Der Metallgehalt der entsprechend den beschriebenen Verfahren durch Tränkung, Sprühtrocknung oder Zerkleinerung im Heißluftstrom und nach thermischer Behandlung erhaltenen oxidischen Katalysatorvorprodukte beträgt 5 bis 18 Gew.-% Ni, 0,25−9,0 Gew.-% Co und 0,10−3,73 Gew.-% Mg.

Die Aktivierung des oxidischen Katalysatorvorproduktes kann stationär, in der Wirbelschicht außerhalb des Methanisierungsreaktors oder im Wirbelschicht-Methanisierungsreaktor »in situ« erfolgen. Die Reduktion erfolgt bei 350 bis 500°C mit reinem Wasserstoff oder Wasserstoff enthaltenden Gasen. Die erfindungsgemäß zusammengesetzten und hergestellten Katalysatoren weisen für die Methanisierung von Kohlenmonoxid insbesondere in der Wirbelschicht überragende mechanische und katalytische Eigenschaften auf.


## Vergleichsbeispiel 1

Für die Herstellung eines kobaltfreien, magnesiumhaltigen Nickel-Tränkungskatalysators werden 247,5 g $Ni(NO_3)_2 \cdot 6 H_2O$ und 65,8 g $Mg(NO_3)_2 \cdot 6 H_2O$ in 130 ml Wasser gelöst. Die Lösung wird auf 80°C erwärmt. In die Lösung werden unter Rühren 100 g $Al_2O_3 \cdot SiO_2$-Trägermaterial (Bezeichnung: L.A.C. der Firma AKZO) eingetragen und die erhaltene Suspension wird 60 Minuten bei 80°C gerührt. Die Weiterverarbeitung durch Filtration, Trocknung und Kalzinierung erfolgt gemäß Beispiel 1. Das kalzinierte Katalysatorvorprodukt hat im wesentlichen die gleiche in Beispiel 1 angegebene Kornverteilung und enthält 13,8 Gew.-% Ni und 1,3 Gew.-% MgO (bezogen auf Gesamtmasse).

Die Reduktion entspricht der Durchführung im Beispiel 1. Die Methanisierung wird in der gleichen Apparatur und unter gleichen Bedingungen gemäß Beispiel 1 durchgeführt.

| | |
|---|---|
| Einsatzgas: | 68,0 Vol.-% $H_2$; 30,8 Vol.-% CO |
| Temperatur in der Reaktionszone: | 410−412°C |
| Reaktionsgemisch (ohne Wasser): | 53,3 Vol.-% $CH_4$; 19,5 Vol.-% $CO_2$; |
| | 23,7 Vol.-% $H_2$; 2,3 Vol.-% CO. |

Beispiel 1

Für die Herstellung eines kobalt- und magnesiumhaltigen Nickel-Tränkungskatalysators werden 247,5 g $Ni(NO_3)_2 \cdot 6 H_2O$, 49,4 g $Co(NO_3)_2 \cdot 6 H_2O$ und 65,8 g $Mg(NO_3)_2 \cdot 6 H_2O$ in 130 ml Wasser gelöst und die Lösung auf 80°C erwärmt. In die Lösung werden unter Rühren 100 g $Al_2O_3 \cdot SiO_2$-Träger (Bezeichnung: L.A.C.-25-C der Firma AKZO) eingetragen und die erhaltene Suspension wird 60 Minuten bei 80°C gerührt. Nach Abtrennung der Tränkungslösung durch Filtration wird der mit den Nitraten des Ni, Co und Mg beladene Feuchtkuchen 16 Stunden bei 110°C getrocknet und das erhaltene Trockenprodukt anschließend 2 Stunden bei 500°C kalziniert.

Es wird ein feinteiliges fluidisierbares Katalysatorvorprodukt mit folgender Kornverteilung erhalten:

| | |
|---|---|
| ≤32 μm | 6,4% |
| >32 − 63 μm | 11,4% |
| >63 − 80 μm | 24,3% |
| >80 −100 μm | 36,6% |
| >100 −125 μm | 18,7% |
| >125 −250 μm | 2,6% |

Das kalzinierte Produkt enthält 11,7 Gew.-% Ni, 2,4 Gew.-% Co und 1,2 Gew.-% MgO, bezogen auf die Gesamtmasse.

Dieses Katalysatorvorprodukt wird in strömendem Wasserstoff in der Wirbelschicht bei einem stündlichen $H_2$-Durchsatz von 1 l $H_2$ pro 1 ml Katalysatorschüttung in 2 Stunden bei 450°C reduziert.

Für die Methanisierung wird ein Gasgemisch (68,0 Vol.-% $H_2$, 30,8 Vol.-% CO, ca. 0,2 Vol.-% $N_2$) eingesetzt. Die Methanisierungsversuche werden mit 20 ml Katalysatorschüttung in einem senkrecht stehenden, elektrisch beheizbaren Rohr mit gasdurchlässigem Boden (Fritte) bei Normaldruck durchgeführt. Die Ofenmanteltemperatur wird auf 380°C eingestellt. Zur Erzeugung eines Wirbelbettes werden 20 l Kohlenmonoxid-Wasserstoffgemisch pro Stunde der obengenannten Zusammensetzung durch die Katalysatorschicht geleitet. In der Wirbelschicht stellt sich eine Temperatur von 412 bis 415°C ein. Das abströmende getrocknete Gasgemisch weist folgende Zusammensetzung auf:

59,8 Vol.-% $CH_4$; 20,2 Vol.-% $CO_2$; 17,9 Vol.-% $H_2$; 0,6 Vol.-% CO und Rest $N_2$.

Durch thermodynamische Gleichgewichtsberechnungen erhält man ausgehend von einem Einsatzgas der oben angegebenen Zusammensetzung ($H_2$/CO = 2,2) für die gemessene Reaktionstemperatur von 415°C und den Gesamtdruck $P_{ges.}$ = 1 bar folgende Gaszusammensetzung (ohne Wasser):

59,4 Vol.-% $CH_4$; 21,9 Vol.-% $CO_2$; 16,9 Vol.-% $H_2$ und 1,3 Vol.-% CO.

Bei der Berechnung wird angenommen, daß keine Kohlenstoffabscheidung stattfindet.

Die weitgehende Übereinstimmung der im Versuch ermittelten Gaszusammensetzung mit der durch thermodynamische Berechnung erhaltenen dokumentiert die hohe Leistungsfähigkeit des erfindungsgemäßen Katalysators.

Vergleichsbeispiel 2

Für die Herstellung eines magnesiumfreien, kobalthaltigen Nickel-Tränkungskatalysators, dessen Trägermaterial dem in Beispiel 1 entspricht, werden 247,5 g $Ni(NO_3)_2 \cdot 6 H_2O$ und 49,5 g $Co(NO_3)_2 \cdot 6 H_2O$ in 130 ml Wasser gelöst. Die Lösung wird auf 80°C erwärmt. In die Lösung werden unter Rühren 100 g $Al_2O_3 \cdot SiO_2$-Trägermaterial (Bezeichnung: L.A.C. der Firma AKZO) eingetragen und die erhaltene Suspension wird 60 Minuten bei 80°C gerührt. Die Weiterverarbeitung durch Filtration, Trocknung und Kalzinierung erfolgt gemäß Beispiel 1. Das kalzinierte Katalysatorvorprodukt hat im wesentlichen die gleiche, in Beispiel 1 angegebene Kornverteilung und enthält 12,3 Gew.-% Ni und 2,2 Gew.-% Co (bezogen auf Gesamtmasse).

Die Reduktion entspricht der Durchführung im Beispiel 1. Die Methanisierung wird in der gleichen Apparatur und unter gleichen Bedingungen gemäß Beispiel 1 durchgeführt.

| | |
|---|---|
| Einsatzgas: | 67,5 Mol.-% CO; 31,6 Vol.-% $H_2$ |
| Temperatur in der Reaktionszone: | 415°C |
| Reaktionsgemisch (ohne Wasser): | 54,1 Vol.-% $CH_4$; 21,2 Vol.-% $CO_2$; |
| | 21,3 Vol.-% $H_2$; 2,4 Vol.-% CO; |
| | ca. 1 Vol.-% $N_2$. |

## Beispiel 2

Für die Herstellung eines kobalt- und magnesiumhaltigen Nickel-Tränkungskatalysators, dessen Trägermaterial im wesentlichen aus $\gamma$-$Al_2O_3$ ($Al_2O_3$ Typ A der Firma Martinswerk) besteht, werden 171,3 g $Ni(NO_3)_2 \cdot 6 H_2O$, 35,1 g $Co(NO_3)_2 \cdot 6 H_2O$ und 44,1 g $Mg(NO_3)_2 \cdot 6 H_2O$ in 95 ml $H_2O$ gelöst. Die Lösung wird auf 80°C erwärmt. In die Lösung (170 ml) werden unter Rühren 120 g des $Al_2O_3$-Trägermaterials eingetragen und die erhaltene Suspension wird 60 Minuten bei 80°C gerührt. Die Weiterverarbeitung durch Filtration, Trocknung und Kalzinierung erfolgt gemäß Beispiel 1. Das kalzinierte Katalysatorvorprodukt hat im wesentlichen die dem Trägermaterial entsprechende Kornverteilung und enthält 8,4 Gew.-% Ni, 1,6 Gew.-% Co und 0,9 Gew.-% Mg (bezogen auf Gesamtmasse).

| | |
|---|---|
| $\leqslant 32$ μm | 10% |
| $> 32 - 63$ μm | 10% |
| $> 63 - 80$ μm | 20% |
| $> 80 - 100$ μm | 27% |
| $> 100 - 125$ μm | 23% |
| $> 125$ μm | 10% |

Die Reduktion entspricht der Durchführung im Beispiel 1. Die Methanisierung wird in der gleichen Apparatur und unter gleichen Bedingungen gemäß Beispiel 1 durchgeführt.

| | |
|---|---|
| Einsatzgas: | 70,5 Mol.-% $H_2$; 28,2 Vol.-% CO |
| Temperatur in der Reaktionszone: | 435°C |
| Reaktionsgemisch (ohne Wasser): | 58,3 Vol.-% $CH_4$; 19,3 Vol.-% $CO_2$; |
| | 21,0 Vol.-% $H_2$; 0,4 Vol.-% CO |
| Thermodynamisch errechneter Wert: | 58,3 Vol.-% $CH_4$ |

## Beispiel 3

Für die Herstellung eines kobalt- und magnesiumhaltigen Nickel-Methanisierungskatalysators, der mittels Sprühtrocknung getrocknet wird, werden 940 g $Ni(NO_3)_2 \cdot 6 H_2O$, 90 g $Co(NO_3)_2 \cdot 6 H_2O$ und 120 g $Mg(NO_3)_2 \cdot 6 H_2O$ in 900 ml Wasser gelöst. Die Lösung wird auf 80°C erwärmt. In die Lösung werden unter Rühren 1200 g $Al_2O_3$ Trägermaterial, das im wesentlichen aus $\gamma$-$Al_2O_3$ besteht ($Al_2O_3$ Typ A der Firma Martinswerk), eingetragen und die erhaltene Suspension wird 30 Minuten bei 80°C gerührt. Anschließend wird die Suspension mit Hilfe eines rotierenden Zerstäubers bei ca. 20 000 Umdrehungen pro Minute in einem auf 260 − 300°C erhitzten Luftstrom versprüht (Sprühtrocknung) und getrocknet.

Die Kalzinierung des Trockenproduktes erfolgt gemäß Beispiel 1. Das kalzinierte Katalysatorvorprodukt hat im wesentlichen die dem Trägermaterial entsprechende Kornverteilung und enthält 12,2 Gew.-% Ni, 1,2 Gew.-% Co und 1,1 Gew.-% Mg (bezogen auf Gesamtmasse).

Die Reduktion entspricht der Durchführung im Beispiel 1. Die Methanisierung wird in der gleichen Apparatur und unter gleichen Bedingungen gemäß Beispiel 1 durchgeführt.

| | |
|---|---|
| Einsatzgas: | 70,5 Vol.-% $H_2$; 29,5 Vol.-% CO Rest $N_2$ |
| Temperatur in der Reaktionszone: | 430°C |
| Reaktionsgemisch (ohne Wasser): | 58,4 Vol.-% $CH_4$; 19,5 Vol.-% $CO_2$; |
| | 20,8 Vol.-% $H_2$; 0,3 Vol.-% CO; |
| | 0,9 Vol.-% $N_2$ |
| Thermodynamisch errechnete Werte: | 58,7 Vol.-% $CH_4$; 19,3 Vol.-% $CO_2$; |
| | 20,6 Vol.-% $H_2$; 0,3 Vol.-% Co |

## Beispiel 4

Für die Herstellung eines kobalt- und magnesiumhaltigen Ni-Methanisierungskatalysators, der mittels eines der Sprühtrocknung verwandten Verfahrens getrocknet wird, werden 79,4 g $Ni(NO_3)_2 \cdot 6\,H_2O$, 8,1 g $Co(NO_3)_2 \cdot 6\,H_2O$ und 10,3 g $Mg(NO_3)_2 \cdot 6\,H_2O$ in 27,4 ml Wasser gelöst. Die Lösung wird auf 80° C erwärmt. In die Lösung werden unter Rühren 134 g $Al_2O_3$ (Typ A der Firma Martinswerk; $\gamma$-$Al_2O_3$) eingebracht, wobei eine feuchte, pastenförmige Masse erhalten wird, die nach 45 Minuten Standzeit mittels eines der Sprühtrocknung verwandten Verfahrens getrocknet wird. Hierbei wird die feuchte, pastenförmige Masse mit Hilfe einer Förderschnecke auf ein Rotorsystem, das im unteren Teil eines zylindrischen Gefäßes installiert ist, aufgebracht. Gleichzeitig wird Heißluft durch das aufgewirbelte Produkt geleitet. Die Kalzinierung erfolgt gemäß Beispiel 1.

Das kalzinierte Katalysatorvorprodukt hat im wesentlichen die dem Trägermaterial entsprechende Kornverteilung und enthält 10 Gew.-% Ni, 1,1 Gew.-% Co und 0,9 Gew.-% Mg (bezogen auf Gesamtmasse).

Die Reduktion entspricht der Durchführung im Beispiel 1. Die Methanisierung wird in der gleichen Apparatur und unter den gleichen Bedingungen gemäß Beispiel 1 durchgeführt.

| | |
|---|---|
| Einsatzgas: | 70,5 Vol.-% $H_2$; 29,5 Vol.-% CO |
| Temperatur in der Reaktionszone: | 430° C |
| Reaktionsgemisch (ohne Wasser): | 58,8 Vol.-% $CH_4$; 19,3 Vol.-% $CO_2$; 20,7 Vol.-% $H_2$; 0,3 Vol.-% CO. |

## Patentansprüche

1. Verfahren zur Herstellung von Methanisierungskatalysatoren, die aus Kobalt, Nickel und einem Trägermaterial bestehen, dadurch gekennzeichnet, daß sie zusätzlich Magnesiumoxid enthalten, das atomare Verhältnis von Nickel, Kobalt und Magnesium von 1 : 0,05 bis 0,5 : 0,05 bis 0,5 beträgt, das Trägermaterial mit thermisch zersetzbaren Salzen des Nickels, Kobalts und Magnesiums imprägniert wird und die aufgetragenen Salze durch Trocknung auf dem Träger fixiert und durch nachfolgende thermische Behandlung in die Metalloxide überführt werden und darauf bei 350 bis 500° C mit reinem Wasserstoff oder Wasserstoff enthaltenden Gasen reduziert wird.

2. Verfahren nach Anspruch 1, dadurch gekennzeichnet, daß als Trägermaterial Aluminiumoxid, Aluminiumoxid-Siliciumdioxid-Kombinationen, z. B. Aluminiumsilicate, Aluminiumoxid-Siliciumdioxid-Mischoxide oder Siliciumdioxid verwendet wird.

3. Verfahren nach Anspruch 2, dadurch gekennzeichnet, daß als Trägermaterial $\gamma$-$Al_2O_3$ oder Aluminiumoxid-Siliciumdioxid-Kombinationen mit 5 bis 30, vorzugsweise 10 bis 20 Gew.-% Aluminiumoxid bezogen auf die gesamte Kombination verwendet wird.

4. Verfahren nach Anspruch 1 bis 3, dadurch gekennzeichnet, daß der Katalysator auf 100 Gew.-Teile Nickelmetall 300 bis 1850 Gew.-Teile, vorzugsweise 400 bis 900 Gew.-Teile Trägermaterial enthält.

5. Verfahren nach Anspruch 1 bis 4, dadurch gekennzeichnet, daß das Trägermaterial eine Oberfläche von 130 bis 600 $m^2$/g, vorzugsweise 150 bis 300 $m^2$/g, besitzt und ein Porenvolumen von mehr als 0,35 $cm^3$/g aufweist.

6. Verfahren nach Anspruch 1 bis 5, dadurch gekennzeichnet, daß die Imprägnierung des Trägermaterials und die Fixierung der aufgebrachten Salze durch Sprühtrocknung erfolgt.

7. Verfahren nach Anspruch 1 bis 5, dadurch gekennzeichnet, daß zur Imprägnierung des Trägermaterials das mit Metallsalzen beladene Trägermaterial in Form einer feuchten pastenförmigen Masse eingesetzt wird, die unter mechanischer Zerteilung und Aufwirbelung in einem heißen Luftstrom getrocknet wird.

## Claims

1. A process for preparing methanation catalysts consisting of cobalt, nickel and a carrier material, characterised in that they additionally contain magnesium oxide, the atomic ratio of nickel, cobalt and magnesium ranges from 1 : 0.05 to 0.5 : 0.05 to 0.5, the carrier material is impregnated with thermally decomposable salts of nickel, cobalt and magnesium and the applied salts are fixed on the carrier by drying and converted into the metal oxides by subsequent thermal treatment and then reduced at 350 to 500° C with pure hydrogen or gases containing hydrogen.

2. A process according to claim 1, characterised in that aluminium oxide, aluminium oxide-silicon dioxide combinations, e.g. aluminium silicates, aluminium oxidesilicon dioxide mixed oxides, or silicon dioxide are used as carrier material.

3. A process according to claim 2, characterised in that $\gamma$-$Al_2O_3$ or aluminium oxide-silicon dioxide combinations with 5 to 30, preferably 10 to 20% by weight of aluminium oxide referred to the total combination, is used as carrier material.

4. A process according to claims 1 to 3, characterised in that the catalyst contains, per 100 parts by weight of nickel metal, 300 to 1850 parts by weight, preferably 400 to 900 parts by weight, of carrier material.

5. A process according to claims 1 to 4, characterised in that the carrier material has a surface of 130 to 600 m²/g, preferably 150 to 300 m²/g, and a pore volume of more than 0.35 cm³/g.

6. A process according to claims 1 to 5, characterised in that the impregnation of the carrier material and the fixing of the applied salts is effected by spray drying.

7. A process according to claims 1 to 5, characterised in that for the impregnation of the carrier material, the carrier material charged with metal salts is used in the form of a moist pasty composition that is dried in a hot air stream under conditions of mechanical dispersion and fluidisation.

## Revendications

1. Procédé pour la fabrication de catalyseurs de méthanisation qui consistent en cobalt, nickel et un matériau de support, caractérisés en ce qu'ils contiennent en outre de l'oxyde de magnésium, les proportions atomiques de nickel, cobalt et magnésium sont de 1 : 0,05 à 0,5 et 0,05 à 0,5, le matériau de support est imprégné avec des sels décomposables thermiquement de nickel, de cobalt et de magnésium et les sels déposés sont fixés sur le support par séchage et transformés en oxydes par traitement thermique ultérieur, et on réduit ensuite à 350−500°C par l'hydrogène pur ou par des gaz contenant de l'hydrogène.

2. Procédé selon la revendication 1, caractérisé en ce qu l'on utilise comme matériau de support l'oxyde d'aluminium, des combinaisons oxyde d'aluminium-dioxyde de silicium, par exemple des silicates d'aluminium, des oxydes mixtes oxyde d'aluminium-dioxyde de silicium ou du dioxyde de silicium.

3. Procédé selon la revendication 2, caractérisé en ce que l'on utilise comme matériau de support $Al_2O_3 \gamma$ ou des combinaisons oxyde d'aluminium-dioxyde de silicium contenant 5 à 30, de préférence 10 à 20, % en poids d'oxyde d'aluminium, par rapport à la combinaison totale.

4. Procédé selon les revendications 1 à 3, caractérisé en ce que le catalyseur contient 300 à 1850 parties en poids de matériau de support, de préférence 400 à 900 parties en poids, pour 100 parties en poids de nickel métallique.

5. Procédé selon les revendications 1 à 4, caractérisé en ce que le matériau de support présente une surface spécifique de 130 à 600 m²/g, de préférence 150 à 300 m²/g, et un volume de pores de plus de 0,35 cm³/g.

6. Procédé selon les revendications 1 à 5, caractérisé en ce que l'imprégnation du matériau de support et la fixation des sels déposés s'effectuent par séchage par pulvérisation.

7. Procédé selon les revendications 1 à 5, caractérisé en ce que l'on utilise pour l'imprégnation le matériau de support chargé en sels métalliques sous forme d'une masse pâteuse humide qui est séchée dans un courant d'air chaud avec broyage mécanique et mise en tourbillons.